# EUROPÄISCHE PATENTSCHRIFT

(11) **EP 1 519 726 B1**
(45) Veröffentlichungstag und Bekanntmachung des Hinweises auf die Patenterteilung: **21.02.2007**
(21) Anmeldenummer: 03762588.6
(22) Anmeldetag: 02.07.2003
(51) Int. Cl.: A61K 31/496, A61P 25/00, C07D 333/70, C07D 307/85, C07D 345/00, C07D 209/42, C07F 17/02

(54) **HETEROARENCARBOXAMIDE ZUR VERWENDUNG ALS DOPAMIN-D3 LIGANDEN ZUR BEHANDLUNG VON ZNS-ERKRANKUNGEN**
UTILIZATION OF HETEROARENE CARBOXAMIDE AS DOPAMINE-D3 LIGANDS FOR THE TREATMENT OF CNS DISEASES
HETEROARENE-CARBOXAMIDES UTILISES COMME LIGANDS DE LA DOPAMINE D3 POUR TRAITER DES MALADIES DU SNC

(30) Priorität: 04.07.2002 DE 10230062; 10.07.2002 DE 10232020
(43) Veröffentlichungstag der Anmeldung: 06.04.2005
(60) Teilanmeldung: 06019977.5
(73) Patentinhaber: SCHWARZ PHARMA AG, 40789 Monheim (DE)
(72) Erfinder: GMEINER, Peter, 91054 Erlangen-Buckenhof (DE); HÜBNER, Harald, 91336 Heroldsbach (DE); SCHLOTTER, Karin, 86732 Oettingen (DE)
(74) Vertreter: HOFFMANN EITLE
(86) Internationale Anmeldenummer: PCT/EP2003/007060
(87) Internationale Veröffentlichungsnummer: WO 2004/004729

(56) Entgegenhaltungen:
- EP-A- 0 343 961
- EP-A- 0 496 692
- WO-A-03/028728
- US-A- 3 646 047
- US-A- 6 090 807
- BETTINETTI, L. ET AL.: "Interactive SAR studies: rational discovery of super-potent and highly selective dopamine D3 receptor antagonists and partial agonists" J. MED. CHEM., Bd. 45, Nr. 21, 6. September 2002 (2002-09-06), Seiten 4594-4597, XP002256409
- LEOPOLDO, M. ET AL.: "Structure-affinity relationship study on N-[4-(4-arylpiperazin-1-yl)butyl]arylcarbo xamides as potent and selective dopamine D3 receptor ligands" J. MED. CHEM., Bd. 45, Nr. 26, 22. November 2002 (2002-11-22), Seiten 5727-5735, XP002256410

## Beschreibung

Dopamin gilt als wichtiger Neurotransmitter des zentralen Nervensystems. Seine Wirkung vermittelt Dopamin durch Bindung an fünf verschiedenen Dopaminrezeptoren. Diese lassen sich aufgrund ihrer Morphologie und ihrer Art der Signalübertragung in die Klassen D1-like (D1 und D5) sowie D2-like (D2-, D3- und D4-Rezeptoren) einteilen (Neve, K.A. *The Dopamine Receptors.* Humana Press, **1997**). Vor allem die Subtypen der D2-Familie spielen bei der Regulation zentralnervöser Vorgänge eine wichtige Rolle. Während die D2-Rezeptoren überwiegend in den Basalganglien exprimiert werden und dort neuromotorische Schaltkreise kontrollieren, finden sich D3-Rezeptoren vor allem im limbischen System, in dem emotionale und kognitive Vorgänge gesteuert werden. Störungen in der Signaltransduktion dieser Rezeptoren führen zu zahlreichen neuropathologischen Situationen. Insbesondere der D3-Rezeptor gilt als vielversprechendes Target für die Entwicklung von Wirkstoffen zur Behandlung von psychiatrischen Erkrankungen wie der Schizophrenie oder der unipolaren Depressionen, von Bewusstseinsstörungen sowie zur Behandlung neurodegenerativer Krankheiten wie dem Parkinsonismus, aber auch zur Behandlung von Drogenabhängigkeit (Pulvirenti, L. et al. *Trends Pharmacol. Sci.* **2002**, *23*, 151-153).

Verbindungen mit Arylpiperazin-Struktur sind bereits als dopaminrezeptoraktive Liganden beschrieben worden (Robarge, M.J. *J. Med. Chem.* **2001**, *44*, 3175-3186). Weiterhin sind Benzamide und Naphthamide mit Arylpiperazin-Partialstruktur als Liganden von Dopaminrezeptoren bekannt (Perrone, R. *J. Med. Chem.* **1998**, *41,* 4903-4909; EP 0 779 284 A1). Kürzlich wurde ein Phenylpiperazinylnaphthamid als selektiver D3-Partialagonist beschrieben, der im Tiermodell hoffnungsvolle Aktivitäten zeigt, die für die Behandlung der Kokainsucht eingesetzt werden könnten (Pilla, M. et al. *Nature* **1999**, *400,* 371-375).

Für wenige Beispiele wurden bisher Arylpiperazinylamide mit sauerstoff-, schwefel- oder stickstoffhaltigen Heteroarensäurekomponenten beschrieben (ES 2027898; EP 343 961; US 3646047; US 3734915). Demgegenüber sind cyanosubstituierte sowie tellurhaltige Derivate und Verbindungen mit Ferrocenylpartialstruktur in der Literatur nicht bekannt.

Wir haben im Rahmen unserer Struktur-Wirkungsuntersuchungen von Dopaminrezeptorliganden neue Verbindungen entdeckt. Diese zeigten bei *in vitro* Untersuchungen hochaffine und hochselektive Bindungseigenschaften am D3-Rezeptor, wie sie bislang noch nicht bekannt sind. Die Verbindungen könnten somit wertvolle Therapeutika zur Behandlung von ZNS-Erkrankungen, wie beispielsweise Schizophrenie, verschiedene Arten der Depression, neurodegenerative Störungen, sexuelle Dysfunktionen sowie der Kokain-, Alkohol-, Opiat- und Nikotinsucht darstellen.

Weiterhin sollten als konkrete Einsatzmöglichkeiten genannt werden: Glaukoma, kognitive Störungen, Restless Leg Syndrom, Hyperaktivitätssyndrom (ADHS), Hyperprolaktinämie, Hyperprolaktinom, Parkinson-assoziierte Bewegungsstörungen, Behandlung von L-DOPA und Neuroleptika induzierten Bewegungsstörungen, z.B. Akathisie, Rigor, Dystonie und Dyskinesien.

Gegenstand dieser Erfindung sind Derivate von 2-Heteroarencarbonsäureamiden mit Arylpiperazinylpartiaistruktur in Form der freien Base und Salze davon, wie sie in Anspruch 1 definiert sind. Sie fallen unter die folgende Formel (I):

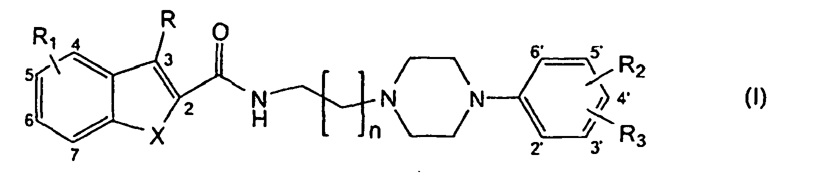

Darin gilt in Formel (I):
- n = 3 und
- R = Wasserstoff
- X = S O, NH oder Te;
- R₁ ist Wasserstoff, Cyano oder Brom;
- R₂ und R₃ sind 2-Methoxy oder 2,3-Dichlorphenyl.

Die Erfindung betrifft insbesondere physiologisch akzeptable Salze der erfindungsgemäßen Verbindungen.

Dem Fachmann ist femer klar, dass je nach Wahl der Substituenten optisch aktive Verbindungen entstehen können. In diesem Fall sind sowohl die Racemate als auch die jeweiligen reinen enantiomeren Formen Gegenstand der vorliegenden Erfindung.

"Physiologisch akzeptable Salze" schließen nicht-toxische Additionssalze einer Base, insbesondere einer Verbindung der Formel (1) in Form der freien Base, mit organischen oder anorganischen Säuren ein. Beispiele für anorganische Säuren schließen HCl, HBr, Schwefelsäure und Phosphorsäure ein. Organische Säuren schließen Essigsäure, Propionsäure, Brenztraubensäure, Buttersäure, α-, β- oder γ-Hydroxybuttersäure, Valeriansäure, Hydroxyvaleriansäure, Capronsäure, Hydroxycapronsäure, Caprylsäure, Caprinsäure, Laurinsäure, Myristinsäure, Palmitinsäure, Stearinsäure, Glykolsäure, Milchsäure, D-Glucuronsäure, L-Glucuronsäure, D-Galacturonsäure, Glycin, Benzoesäure, Hydroxybenzoesäure. Gallussäure, Salicylsäure, Vanillinsäure, Cumarsäure, Kaffeesäure, Hippursäure, Orotsäure, L-Weinsäure, D-Weinsäure, D,L-Weinsäure, meso-Weinsäure, Fumarsäure, L-Äpfelsäure, D-Äpfelsäure, D,L-Äpfelsäure, Oxalsäure, Malonsäure, Bernsteinsäure, Maleinsäure, Oxalessigsäure, Glutarsäure, Hydroxyglutarsäure, Ketoglutarsäure, Adipinsäure. Ketoadipinsäure, Pimelinsäure, Glutaminsäure, Asparaginsäure, Phthalsäure, Propantricarbonsäure, Zitronensäure, Isozitronensäure, Methansulfonsäure, Toluolsulfonsäure und Trifluormethansulfonsäure ein.

Als bevorzugte Strukturen sind Verbindungen der Formel (I) zu nennen, in denen X durch NH, S oder O dargestellt wird.

Die folgenden Verbindungen stellen konkrete Ausführungsformen der erfindungsgemäßen Verbindungen dar:
(B18): N-4-(4-(2-Methoxyphenyl)piperazin-1-yl)butyl-5-cyan-2-benzo[b]thiophenylcarbamid
(B19): N-4-(4-(2,3-Dichlorphenyl)piperazin-1-yl)butyl-5-cyan-2-benzo[b]thiophenylcarbamid
(B20): N-4-(4-(2-Methoxyphenyl)piperazin-1-yl)butyl-6-cyan-2-benzo[b]thiophenylcarbamid
(B21): N-4-(4-(2,3-Dichlorphenyl)piperazin-1-yl)butyl-6-cyan-2-benzo[b]thiophenylcarbamid
(B1): N-4-(4-(2-Methoxyphenyl)piperazin-1-yl)butyl-2-benzo[b]thiophenylcarbamid
(B2): N-4-(4-(2,3-Dichlorphenyl)piperazin-1-yl)butyl-2-benzo[b]thiophenylcarbamid
(B22): N-4-(4-(2-Methoxyphenyl)piperazin-1-yl)butyl-5-brom-2-benzo[b]thiophenylcarbamid
(B23): N-4-(4-(2,3-Dichlorhenyl)piperazin-1-yl)butyl-5-brom-2-benzo[b]thiophenylcarbamid
(B10): N-4-(4-(2,3-Dichlorphenyl)piperazin-1-yl)butyl-2-indolylcarbamid
(B11): N-4-(4-(2-Methoxyphenyl)piperazin-1-yl)butyl-5-cyan-2-indolylcarbamid
(B12): N-4-(4-(2-Methoxyphenyl)piperazin-1-yl)butyl-5-brom-2-indolylcarbamid
(B13): N-4-(4-(2-Methoxyphenyl)piperazin-1-yl)butyl-6-cyan-2-indolylcarbamid
(B14): N-4-(4-(2,3-Dichlorphenyl)piperazin-1-yl)butyl-5-brom-2-indolylcarbamid
(B15): N-4-(4-(2,3-Dichlorphenyl)piperazin-1-yl)butyl-6-cyan-2-indolylcarbamid
(B25): N-4-(4-(2,3-Dichlorphenyl)piperazin-1-yl)butyl-5-cyan-2-indolylcarbamid
(B7): N-4-(4-(2-Methoxyphenyl)piperazin-1-yl)butyl-5-cyan-2-benzo[b]furanylcarbamid
(B3): N-4-(4-(2-Methoxyphenyl)piperazin-1-yl)butyl-2-benzo[b]furanylcarbamid
(B4): N-4-(4-(2,3-Dichlorphenyl)piperazin-1-yl)butyl-2-benzo[b]furanylcarbamid
(B5): N-4-(4-(2-Methoxyphenyl)piperazin-1-yl)butyl-5-brom-benzo[b]furanylcarbamid
(B6): N-4-(4-(2,3-Dichlorphenyl)piperazin-1-yl)butyl-5-brom-2-benzo[b]furanyl-carbamid
(B8): N-4-(4-(2-Methoxyphenyl)piperazin-1-yl)butyl-2-benzo[b]tellurophenylcarbamid
(B9): N-4-(4-(2,3-Dichlorphenyl)piperazin-1-yl)butyt-2-benzo[b]tellurophenylcarbamid
sowie pharmazeutisch akzeptable Salze dieser Verbindungen.

Die erfindungsgemäßen Verbindungen sind zum therapeutischen Einsatz als Dopamin D3-Liganden geeignet. Besonders bevorzugt sind Verbindungen der allgemeinen Formel (I) oder pharmazeutisch akzeptable Salze davon, worin X = NH, S oder O ist.

Der Begriff "hochaffine D3-Liganden" umfasst Verbindungen, die in einem Radioligandexperiment Bindung an humane Dopamin D3-Rezeptoren mit einem Ki-Wert von vorzugsweise nicht mehr als 10 nM, besonders bevorzugt nicht mehr als 1 nM zeigen (vgl. Hübner, H. et al. *J. Med. Chem.* **2000**, *43*, 756-762 sowie nachfolgender Abschnitt "Biologische Aktivität").

Ein Aspekt der vorliegenden Erfindung betrifft selektive D3-Liganden. Der Begriff "selektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 10 niedriger als für mindestens fünf der sieben folgenden Rezeptoren ist: Dopamin-Rezeptoren D1, D2long, D2short und D4.4, Serotonin-Rezeptoren 5-HT1A und 5-HT2 und Alpha 1 Adrenozeptor.

Ein anderer Aspekt der Erfindung betrifft hochselektive Dopamin D3-Liganden. Der Begriff "hochselektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 100 niedriger als für mindestens drei, bevorzugt für alle der Dopamin-Rezeptoren D 1, D2long, D2short und D4.4 ist.

D3-Liganden können am D3-Rezeptor agonistische, antagonistische oder partialagonistische Wirkung haben. Die entsprechenden intrinsischen Aktivitäten der erfindungsgemäßen Verbindungen lassen sich in Mitogeneseassays messen, wie in der Literatur beschrieben (Hübner, H. et al. *J. Med. Chem.* **2000**, *43*, 4563-4569 und Löber, S. *Bioorg. Med. Chem. Lett.* **2002**, 12.17, 2377-2380). In Abhängigkeit von der Pathophysiologie der zugrunde liegenden Erkrankung kann therapeutisch eine stärker agonistische, stärker antagonistische oder eine partialagonistische Aktivität gewünscht sein. Die vorliegende Erfindung erlaubt daher in exzellenter Weise eine Feineinstellung der gewünschten Aktivität.

Ein weiterer Gegenstand der Erfindung ist daher ein Arzneimittel, das eine oder mehrere der konkret aufgeführten Verbindungen wie oben definiert, gegebenenfalls in Form eines pharmazeutisch akzeptablen Salzes, enthält. Bevorzugt sind darin eine oder mehrere der Verbindungen der allgemeinen Formel (I) oder pharmazeutisch akzeptable Salze enthalten, worin X = NH, S oder O ist.

Die Erfindung betrifft ebenfalls die Verwendung einer oder mehrerer der konkret aufgeführten Verbindungen, gegebenenfalls in Form eines pharmazeutisch akzeptablen Salzes, zur Behandlung, einschließlich Therapie und Prophylaxe, der hier genannten Indikationen sowie zur Herstellung eines Arzneimittels für die hier genannten Indikationen.

Bevorzugt werden zur Herstellung von Arzneimitteln solche erfindungsgemäßen Verbindungen ausgewählt, die selektive D3-Liganden sind. Besonders bevorzugt werden hochselektive D3-Liganden verwendet.

Die erfindungsgemäßen Verbindungen haben Potential in der Therapie oder Prophylaxe einer Reihe von Erkrankungen, die insbesondere mit einer Störung des Dopaminstoffwechsels oder der dopaminergen Signalkaskade einhergehen.

Beispiele für solche Erkrankungen sind Kokain-, Alkohol-, Opiat- und Nikotinsucht; neurodegenerative Störungen, insbesondere Morbus Parkinson; sexuelle Dysfunktion, insbesondere männliche erektile Dysfunktion; Depression, insbesondere endogene monophasische Depression ("major depression") und Schizophrenie.

Weitere Beispiele, die der Therapie oder Prophylaxe mit den erfindungsgemäßen Verbindungen zugänglich sind, sind Hyperprolaktinämie; Hyperprolaktinom; Glaucoma; kognitive Störungen; Restless Leg Syndrom; Hyperaktivitätssyndrom (ADHS); Parkinson-assoziierte Bewegungsstörungen, z.B. Rigor, Dystonie und Dyskinesie; L-Dopa-induzierte Störungen, z.B. Angstzustände, Schlafstörungen, Psychosen, Dyskinesien und Dystonien; idiopathische Dystonien, insbesondere Segawa-Syndrom; Neuroleptika-induzierte (tardive) Dyskinesie, Dystonie und Akathisie.

Insbesondere sind die erfindungsgemäßen Verbindungen zur Herstellung eines Arzneimittels zur Behandlung Dopa-sensitiver Bewegungsstörungen geeignet. Solche Bewegungsstörungen können beispielsweise Dyskinesien, Dystonien, Rigor und Tremor sein. Unter dem Begriff "Dopa-sensitiv" wird dabei verstanden, dass die Bewegungsstörung günstig durch Gabe von Arzneimitteln beeinflusst werden kann, die die dopaminerge Signalübertragung beeinflussen. Ein typisches Beispiel hierfür ist das Segawa-Syndrom, eine idiopathische Dystonie, bei der das Ansprechen auf L-Dopa als diagnostisches Kriterium genutzt werden kann.

Eine bevorzugte Verwendung betrifft die Herstellung eines Arzneimittels zur Behandlung von Dyskinesien und Dystonien, die spontan im Zuge von Parkinson-Erkrankungen auftreten können, aber auch Medikamenten-induziert sein können. Unter den Medikamenten-induzierten Dyskinesien und Dystonien sind insbesondere solche zu nennen, die durch Neuroleptika bzw. Dopaminantagonisten oder Dopaminagonisten oder L-Dopa induziert wurden.

Ferner können die Arzneimittel zum Medikamenten-unterstützten Abstillen nach Schwangerschaften eingesetzt werden.

Schließlich können die erfindungsgemäßen Arzneimittel in Abhängigkeit von der zu behandelnden Erkrankung auch als Kombinationspräparat zur gleichzeitigen oder sequentiellen Gabe ausgebildet sein.

Beispielsweise kann eine Verkaufseinheit, die eine zur Behandlung der Parkinson-Erkrankung enthaltende L-Dopa Medikation enthält, auch eine pharmazeutische Zusammensetzung umfassen, die eine der erfindungsgemäßen Verbindungen mit z.B. hochselektivem, partial-agonistischem Wirkprofil enthält. Dabei können L-Dopa und die erfindungsgemäße Verbindung in der gleichen pharmazeutischen Formulierung, z.B. einer Kombinationstablette, oder auch in unterschiedlichen Applikationseinheiten vorliegen, z.B. in Form zweier separater Tabletten. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikationsschemata denkbar sind, die alle Gegenstand der Erfindung sind.

Eine Ausführungsform der Erfindung betrifft daher ein Arzneimittel, das L-Dopa oder ein Neuroleptikum sowie eine erfindungsgemäße Verbindung zur gleichzeitigen oder zeitlich aufeinanderfolgenden Verabreichung an den Patienten enthält.

Üblicherweise bestehen die erfindungsgemäßen Arzneimittel aus einer pharmazeutischen Zusammensetzung, die neben den erfindungsgemäßen D3-Liganden, wie oben beschrieben, mindestens einen pharmazeutisch annehmbaren Träger oder Hilfsstoff enthält.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur intravenösen, intramuskulären, intrakutanen, subkutanen, oralen, bukkalen, sublingualen, nasalen, transdermalen, inhalativen, rektalen oder intraperitonealen Verabreichung angepasst sein.

Entsprechende Formulierungen und hierfür geeignete pharmazeutische Träger bzw. Hilfsstoffe, wie Füllstoffe, Sprengmittel, Bindemittel, Gleitmittel, Stabilisatoren, Aromastoffe, Antioxidantien, Konservierungsmittel, Dispersions- oder Lösungsmittel, Puffer oder Elektrolyte, sind dem Fachmann auf dem Gebiet der Pharmazeutik bekannt und sind beispielsweise in Standardwerken wie Sucker, Fuchs und Speiser ("Pharmazeutische Technologie", Deutscher Apotheker Verlag, 1991) und Remington ("The Science and Practice of Pharmacy", Lippincott, Williams & Wilkins, 2000) beschrieben.

In einer bevorzugten Ausführungsform der Erfindung werden die pharmazeutischen Zusammensetzungen, die die erfindungsgemäßen Verbindungen enthalten, oral verabreicht und können beispielsweise als Kapsel, Tablette, Pulver, Granulat, Dragee oder in flüssiger Form vorliegen.

Dabei kann die Formulierung als schnell freisetzende Darreichungsform ausgestaltet sein, wenn ein rascher Wirkeintritt gewünscht ist. Entsprechende orale Formulierungen sind beispielsweise beschrieben in EP 0 548 356 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt.

Alternative pharmazeutische Zubereitungen können beispielsweise Infusions- oder Injektionslösungen. Öle, Suppositorien, Aerosole, Sprays, Pflaster, Mikrokapseln oder Mikropartikel sein.

Eine bevorzugte Verbindung zur Herstellung der erfindungsgemäßen Arzneimittel, insbesondere zur Behandlung L-Dopa-indiuzierter Dyskinesien, ist die folgende Verbindung:
(B24): N-4-(4-(2-Methoxyphenyl)piperazin-1-yl)butyl-2-indolylcarbamid

Die Verbindungen wurden nach den Methoden entsprechend der Literatur (Glennon, R.A et al. *J. Med. Chem.* **1988**, *31*, 1968-1971) hergestellt.

Dazu wurden die Säurederivate vom Typ (A), die entweder käuflich zugänglich waren oder nach Literaturvorschriften synthetisiert wurden, in Form ihrer Carbonsäurechloride aktiviert und mit der freien Base vom Typ (B) zu den Derivaten der Formel (I) umgesetzt: 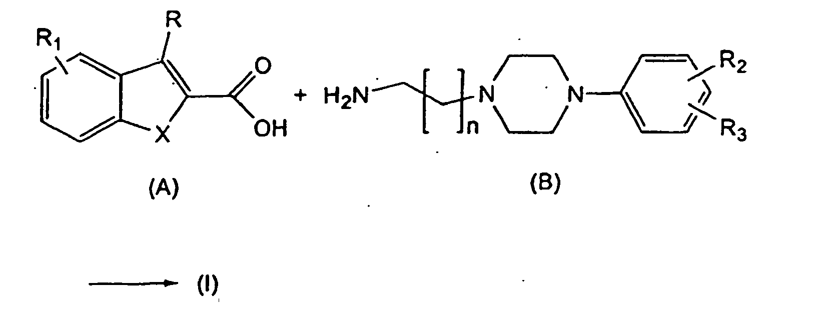 worin n, R, R₁, R₂ und R₃ wie oben für (I) definiert sind.

Alternativ zur oben erwähnten Methode der Aktivierung der Säurederivate können auch andere Reaktionen eingesetzt werden, wie zum Beispiel die Aktivierung von Säuren durch Hydroxyazabenzotriazol (Kienhöfer, A. *Synlett* **2001,** 1811-1812). Beispielsweise können die Verbindungen der Formel (II) durch Aktivierung von Ferrocen-2-carbonsäure mit HATU und anschließende Reaktion mit den Basen des Typs (B) hergestellt werden.

Für die Herstellung der Arylpiperazinylamine vom Typ (B) können z.B. käuflich zugängliche 2-Methoxy- bzw. 2,3-Dichlorphenylpiperazine mit Brombutylphthalimid in Xylol alkyliert werden. Anschließende Hydrazinolyse der phthalimidsubstituierten Strukturen liefert die primären Amine vom Typ (B). Dies wird anhand des folgenden exemplarischen Reaktionsschemas verdeutlicht:

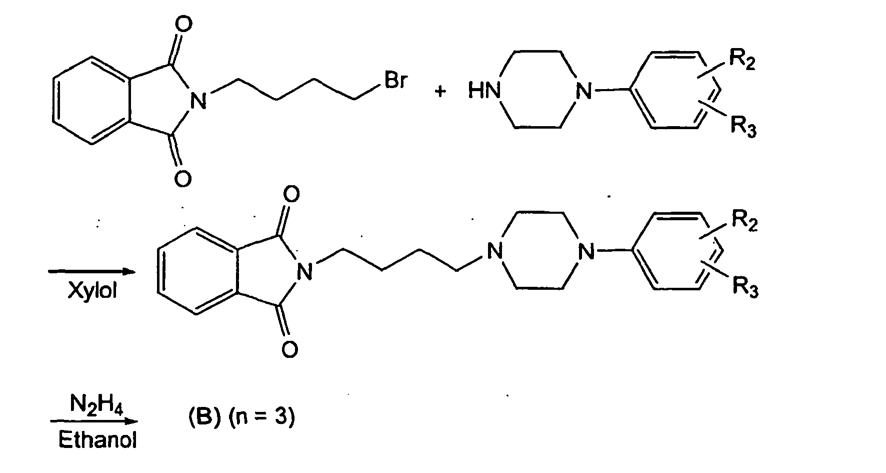

### BEISPIELE

### Synthese der Amine vom Typ (B)

### 4-(4-(2,3-Dichlorphenyl)piperazin-1-yl)butylamin

2,3 g (10 mmol) freie Base des 2,3-Dichlorphenylpiperazins werden in 10 ml Xylol gelöst und auf 70°C erhitzt. Dann werden 1,4 g (5 mmol) 4- Brombutylphthalimid in 20 ml Xylol gelöst and langsam der Lösung des Piperazins zugetropft. Das Reaktionsgemisch wird für 24 Stunden bei 125°C erhitzt. Nach Abkühlen der Mischung auf 0°C wird abfiltriert and das Filtrat evaporiert. Das entstandene N-4-(4-(2,3-Dichlorphenyl)piperazin-1-yl)butylphthalimid wird durch Flashchromatographie an SiO₂ mit Ethylacetat gereinigt.
Ausbeute: 4,0 g (= 92%)

Zu einer Suspension von N-4-(4-(2,3-Dichlorphenyl)piperazin-1-yl)butylphthalimid in 40 ml Ethanol wird eine Lösung von 80% Hydrazinhydrat (0,45 ml, 2,5 eq) in 5 ml Ethanol zugetropft. Die Mischung wird für 3 Stunden unter Rückfluss erhitzt, anschließend auf RT abgekühlt, der dabei ausfallende Feststoff abfiltriert, und die ethanolische Lösung im Vakuum abgedampft.

Reinigung durch Flashchromatographie mit CH₂Cl₂-MeOH-Me₂EtN:90-8-2 liefert die Base 4-(4-(2,3-Dichlorphenyl)piperazin-1-yl)butylamin mit einer Ausbeute von 900 mg (= 60 %).

MS: m/z 301(M+); IR: (NaCl): 3397; 2939; 2817; 1641; 1572; 1500; 1482; 1452; 1376; 1240; 1152; 1118; 1023; 917; 791; 749; 698; 661.1H-NMR (CDCl₃, 360 MHz) δ (ppm): 1,48-1,64 (m, 4H,CH₂-CH₂); 2,41-2,46 (t, J = 7,6, 2H, CH₂N); 2,64 (m, 4H, pip); 2,72-2,76 (m, 2H, CH₂NCO); 3,07 (m, 4H, pip); 6,93-6,99 (m, 1H, H₅, Phenyl); 7,11-7,17 (m, 2H, H₄, H₆, Phenyl).

### Beispiel 1

### N-4-(4-(2-Methoxyphenyl)piperazin-1-yl)butyl-2-benzo[b]thiophenylcarbamid

0,4 mmol Benzothiophen-2-carbonsäure (0,071 g) werden in 4 ml trockenem Toluol und 4 ml trockenem Chloroform gelöst. Es werden 0,02 ml trockenes DMF and 0,11 ml (1,51 mmol) SOCl₂ zugegeben. Es wird für 30 Minuten im Ölbad auf 90°C erhitzt. Anschließend wird das Lösungsmittel abrotiert und im Feinvakuum getrocknet. Das Säurechlorid wird in 4 ml Chloroform gelöst und unter Rühren bei 0°C zu einer Lösung aus 0,4 mmol 4-(4-(2-Methoxyphenyl)piperazin-1-yl)butylamin (0,105 g) und 0,17 ml Et₃N in 5 ml Chloroform hinzugefügt. Nach 15 Std. Reaktionszeit wird mit wässriger NaHCO₃-Lösung gewaschen, das organische Lösungsmittel mit MgSO₄ getrocknet und im Vakuum abgedampft. Reinigung durch Flash-Chromatographie an Kieselgel mit CH₂Cl₂-MeOH:9-1 ergibt 114 mg (68 % Ausbeute über 2 Reaktionsschritte) von N-4-(4-(2-Methoxyphenyl)piperazin-1-yl)butyl-2-benzo[b]thiophenylcarbamid.

Smp.: 147°C; MS: m/z 423 (M⁺); IR (NaCl): 3316; 2938; 2817; 1735; 1629; 1544; 1500; 1241; 1026; 731. ¹H-NMR (CDCl₃, 360 MHz) δ (ppm):1,65-1,74 (m, 4H, CH₂-CH₂); 2,47 (t, 2H, CH₂N, J=6,7 Hz); 2,65 (m, 4H, pip); 3,08 (m, 4H, pip); 3,48-3,53 (m, 2H, CH₂NCO), 3,85 (s, 3H, OCH₃); 6,72 (t, 1H, NH, J= 4,3 Hz); 6,84-7,01 (m, 4H, Phenyl); 7,36-7,44 (m, 2H, H₅, H₆); 7,76 (s, 1H, H₃); 7,79-7,85 (m, 2H, H₇, H₄). ¹³C-NMR (CDCl₃, 90 MHz) δ (ppm): 162,4; 152,3; 141,2; 140,7; 139,1; 138,7; 126,2; 125,0; 124,9; 122,9; 122,7; 120,9; 118,2; 111,2; 57,9; 55,3; 53,4; 50,5; 40,0; 27,4; 24,3.
C H N (%): C₂₄H₂₉N₃O₂S;
Ber.: C 68,05; H 6,90; N 9,92; S 8,15; Gef.: C 68,11; H 6,95; N 9,93; S 8,09.

### Beispiel 2

### N-4-(4-(2,3-Dichlorphenyl)piperazin-1-yl)butyl-2-benzo[b]thiophenylcarbamid

### Synthese analog zu Beispiel 1.

### Ausbeute: 126 mg (68% über 2 Reaktionsschritte)

Smp.: 153°C. MS: m/z 462 (M⁺). IR (NaCl): 3298; 2967; 2934; 2809; 1640; 1599; 1576; 1530; 1442; 1420; 1301; 1167; 1131; 962; 882; 808; 781; 712. ¹H-NMR: (CDCl₃, 360 MHz) δ (ppm): 1,63-1,76 (m, 4H, CH₂-CH₂); 2,48 (t, J=6,9 Hz, 2H, CH₂N); 2,66 (m, 4H, pip); 3,05 (m, 4H, pip); 3,49-3,54 (m, 2H, CH₂NCO); 6,79 (br,t, J=5,3 Hz, 1 H, NH); 6,84-6,86 (dd, J =1,6 Hz, J =7,5 Hz, 1H, Phenyl); 7,08-7,16 (m, 2H, Phenyl); 7,37-7,44 (m, 2H, H₅, H₆); 7,77-7,78 (s, 1H, H₃); 7,80-7,90 (m; 2H; H₄, H₇). ¹³C-NMR (CDCl₃, 90 MHz) δ (ppm): 157,9; 156,1; 152,3; 151,1; 141,2; 129,9; 127,8; 123,0; 121,0; 118,2; 113,0; 112,3; 106,6; 109,7; 107,9; 57,9; 53,5; 50,5; 39,4; 27,4; 24,3.
C H N (%): C₂₃H₂₅Cl₂N₃OS
Ber.: C 60,25; H 6,11; N 8,78; Gefunden: C 59,94; H 6,04; N 8,81.

### Beispiel 3

### N-4-(4-(2-Methoxyphenyl)piperazin-1-yl)butyl-2-benzo[b]furanylcarbamid

### Synthese analog zu Beispiel 1.

### Ausbeute: 75,2 mg (46% Ausbeute über 2 Reaktionsschritte)

Smp.: 121°C. MS: m/z 431 (M⁺). IR (NaCl): 3311,2; 3060; 2937; 2815; 2216; 1654; 1592; 1500; 1321; 1240; 1178; 1145; 748. ¹H-NMR (CDCl₃, 360 MHz) δ (ppm): 1,67-1,74 (m, 4H, CH₂-CH₂); 2,49 (t, 2H, CH₂N, J=6,9 Hz); 2,69 (m, 4H, pip); 3,13 (m, 4H, pip); 3,56-3,50 (m, 2H, CH₂NCO); 3,86 (s, 3H, OCH₃); 6,85-6,87 (m, 1H, Phenyl); 6,90-6,93 (m, 2H Phenyl); 6,99-7,02 (m, 2H, Phenyl und NH); 7,26-7,31 (m, 1H, H₅); 7,37-7,42 (m, 1H, H₆); 7,46-7,48 (m, 2H, H₄, H₃); 7,77-7,79 (m, 1H, H₇). ¹³C-NMR (CDCl₃, 90 MHz) δ (ppm): 158,9; 154,7; 152,3; 148,9; 141,3; 127,7; 126,7; 123,6; 122,9; 122,7; 120,9; 118,2; 111,7; 111,2; 110,2; 58,0; 55,3; 53,5; 50,5; 39,2; 27,5; 24,3.
C H N (%): C₂₄H₂₉N₃O₃·0,3 H₂O;
Ber.: C 69,81; H 7,23; N 10,18; Gef,: C 69,84; H 7,33; N 10,21.

### Beispiel 4

### N-4-(4-(2,3-Dichlorphenyl)piperazin-1-yl)butyl-2-benzo[b]furanylcarbamid

### Synthese analog zu Beispiel 1.

### Ausbeute: 105 mg (58 % Ausbeute über 2 Reaktionsschritte)

Smp.: 150°C. MS: m/z 446 (M+). IR (NaCl): 3310; 2939; 2819; 1652; 1595; 1577; 1520; 1448; 1421; 1299; 1257; 1241; 1176; 1141; 1044; 960; 908; 780; 748; 713, 669, 645. ¹H-NMR (CDCl₃, 360 MHz) δ (ppm): 1,67-1,75 (m, 4H, CH₂-CH₂); 2,52 (t, J=6,7 Hz, 2H, CH₂N); 2,69 (m, 4 H, pip); 3,13 (m, 4H, pip); 3,51-3,56 (m, 2H, CH₂NCO); 6,92-6,95 (dd, J=2,3 Hz, 7,3 Hz, 1H, Phenyl); 7,00 (brt, J= 4,3 Hz, 1 H, NHCO); 7,10-7,17 (m, 2H, Phenyl); 7,26-7,31 (m, 1H, H₄); 7,38-7,43 (m, 1H, H₆); 7,46-7,48 (m, 2H, H₃, H₅): 7,66-7,68 (m; 1H, H₇). ¹³C-NMR (CDCl₃, 90 MHz) δ (ppm): 158,9; 154,7; 151,2; 148,2; 134,0; 127,7; 127,5; 127,4; 126,8; 124,6; 123,7; 122,7; 118,6; 111,6; 110,2; 57,9; 53,3; 51,1; 39,2; 27,5; 24,2.
CHN(%): C₂₃H₂₅Cl₂N₃O₂
Ber.: C 61,89; H 5,65; N 9,41; Gefunden: C 61,74; H 5,86; N 9,05.

### Beispiel 5

### N-4-(4-(2-Methoxyphenyl)piperazin-1-yl)butyl-5-brom-2-benzo[b]furanylcarbamid

Synthese analog zu Beispiel 1. Dabei erfolgte die Herstellung von 5-Brom-2-benzo[b]furanylcarbonsäure nach Literatur (Dann, O. *Liebigs Ann. Chem.* **1986**, 438-455). Ausbeute: 107,8 mg (56% Ausbeute über 2 Reaktionsschritte)

Smp.: 124°C. MS m/z 485 (M⁺). IR (NaCl): 3450,0; 3289.9; 3068.2; 2927.4; 2765; 1650; 1567; 1535; 1500; 1438; 1238; 1178; 1022; 802; 748. ¹H-NMR (CDCl₃, 360 MHz) δ (ppm): 1,67-1,74 (m, 4H, CH₂-CH₂); 2,49 (t, J=6,9 Hz, 2H, CH₂N); 2,69 (m, 4H, pip); 3,13 (m, 4H, pip); 3,56-3,60 (m, 2H, CH₂NCO); 3,86 (s, 3H, OCH₃); 6,85-6,87 (m, 1H, Phenyl); 6,91-6,93 (m, 2H, Phenyl); 6,97-7,00 (m, 1H, Phenyl); 7,00 (brt, J=4,8 Hz, 1H, NH); 7,32-7,35 (d, J= 8,9 Hz, 1H, H₄); 7,38-7,39 (m, 1H, H₃); 7,48 (dd, J=8,7 Hz, J=2,0 Hz, 1H, H₆); 7,79-7,80 (m, 1H, H₇). ¹³C-NMR (CDCl₃, 90 MHz) δ (ppm): 158,4; 153,3; 152,3; 150,1; 141,2; 129,7; 129,6; 125,2; 122,9; 120,9; 120,9; 118,2; 116,7; 113,1; 111,2; 109,4; 64,8; 57,9; 55,3; 53,5; 50,4; 39,3; 27,5; 24,3.
CHN (%): C₂₄H₂₉BrN₃O₂;
Ber.: C 59,26; H 5,80; N 8,64; Gef.: C 59,05; H 5,81; N 8,68.

### Beispiel 6

### N-4-(4-(2,3-Dichlorphenyl)piperazin-1-yl)butyl-5-brom-2-benzo[b]furanylcarbamid

### Synthese analog zu Beispiel 5.

### Ausbeute:102 mg (47 % Ausbeute über 2 Reaktionsschritte)

Smp.: 145°C. MS m/z 524 (M⁺); IR (NaCl): 3400; 2937; 2815; 2227; 1666; 1594; 1527; 1500; 1294; 1240; 1141; 1118; 1025; 842; 746. ¹H-NMR (CDCl₃, 360 MHz) δ (ppm): 1,63-1,74 (m, 4H, CH₂-CH₂); 2,49-2,52 (t, J=6,7 Hz, 2H, CH₂N); 2,68 (m, 4H, pip); 3,09 (m, 4H, pip); 3,49-3,56 (m, 2H, CH₂NCO); 6,92-6,94 (dd, J=2,1 Hz, J=7,5 Hz, 1H, Phenyl); 6,98-7,01 (brt, J=3,0 Hz, 1 H, NH); 7,33-7,36 (d, J = 5,3 Hz, 1H, H₄); 7,39 (m, 1 H, H₃); 7,48-7,51 (dd, J=8,7 Hz, J=2,0 Hz, 1 H, H₆); 7,80-7,81 (d, J=2,0 Hz; 1 H, H₇). ¹³C-NMR (CDCl₃, 90 MHz) δ (ppm): 157,9; 156,1; 152,3; 151,1; 141,2; 129,9; 127,8; 123,0; 121,0; 118,2; 113,0; 112,3; 106,6; 109,7; 107,9; 57,9; 55,4; 53,5; 50,5; 39,4; 27,4; 24,3; 21,0.
C H N(%): C₂₃H₂₄BrCl₂N₃O₂
Ber.: C 52,57; H 4,67; N 8,03; Gef.: C 52,63; H 4,67; N 8,03.

### Beispiel 7

### N-4-(4-(2-Methoxyphenyl)piperazin-1-yl)butyl-5-cyan-2-benzo[b]furanylcarbamid

0,37 mmol (141 mg) HATU und 0,37 mmol (69 mg) der 5-Cyano-2-benzo[b]furanylcarbonsäure (Dann, O. *Liebigs Ann. Chem.* **1986**, 438-455) werden bei 0°C in 1 ml DMF gelöst und 0,74 mmol (0,13 ml) DIEA zugegeben. Anschließend wird 0,33 mmol (87 mg) 4-(4-(2-Methoxyphenyl)piperazin-1-yl)butylamin in DMF gelöst and bei 0°C zu der Reaktionslösung zugetropft. Nach 1 Stunde wird der Reaktionsansatz in CHCl₃ aufgenommen und mit NaHCO₃-Lösung sowie Wasser gewaschen. Nach Trocknung mit MgSO₄ wird das Lösungsmittel abgedampft und durch Flashchromatographie (SiO₂; Petrolether-Ethylacetat: von 1-1 nach Ethylacetat) gereinigt. Ausbeute: 41 mg (28%)

Smp.: 96°C. MS m/z 432 (M⁺). IR (NaCl): 3400; 2937; 2815; 2227; 1666; 1594; 1527; 1500; 1294; 1240; 1141; 1118; 1025; 842; 746. ¹H-NMR (CDCl₃, 360 Mhz) δ (ppm): 1,67-1,74 (m, 4H, CH₂-CH₂); 2,49 (t, J=6,9 Hz, 2H, CH₂N); 2,69 (m, 4H, pip); 3,13 (m, 4H, pip); 3,56-3,60 (m, 2H, CH₂NCO); 3,86 (s, 3H, OCH₃); 6,84-7,02 (m, 4H, Phenyl); 7,12-7,15 (brt, J=5,1 Hz, 1H, NH); 7,50-7,51 (m, 1H, H₄); 7,55-7,57 (m, 1H, H₃); 7,65-7,68 (m ,1H, H₆); 8,03-8,04 (m, 1H, H₇). ¹³C-NMR (CDCl₃, 90 MHz) δ (ppm): 157,9; 156,1; 152,3; 151,1; 141,2; 129,9; 127,8; 123,0; 121,0; 118,2; 113,0; 112,3; 106,6; 109,7; 107,9; 57,9; 55,4; 53,5; 50,5; 39,4; 27,4; 24,3; 21,0.

### Beispiel 8

### N-4-(4-(2-Methoxyphenyl)piperazin-1-yl)butyl-2-benzo[b]tellurophenylcarbamid

### Synthese analog zu Beispiel 7.

### Ausbeute: 73 mg (58 %)

Smp.: 85°C. MS m/z 521 (M⁺). IR (KBr): 3320; 3047; 2933; 2815; 1616; 1566; 1541; 1375; 1498; 1240; 1023; 748. ¹H-NMR (CDCl₃, 360 MHz) δ (ppm): 1,62-1,66 (m, 4H, CH₂-CH₂); 2,42-2,45 (t, J= 6,7 Hz, 2H, CH₂N); 2,63 (m, 4H, pip); 3,04 (m, 4H, pip); 3,38-3,43 (m, 2H, CH₂NCO); 3,78 (s, 3H, OCH₃); 6,70-6,76 (brt, J= 4,3 Hz, 1H, NH); 6,77-6,83 (m, 3H, Phenyl); 6,94-6,96 (m, 1H, Phenyl); 7,09-7,14 (m, 1H, H₆); 7,28-7,32 (m, 1 H, H₅); 7,74-7,76 (d, J=7,5 Hz, 1H, H₄); 7,84-7,86 (d, J =7,8 Hz, 1H, H₇); 8,13 (s, 1H, H₃). ¹³C-NMR (CDCl₃, 90 MHz) δ (ppm): 166,1; 152,3; 147,9; 141,2; 140,2; 134,8; 132,3; 129,3; 125,8; 125,5; 122,9; 121,0; 118,2; 111,2; 57,9; 55,3; 53,3; 50,5; 40,4; 27,4; 24,4.

### Beispiel 9

### N-4-(4-(2,3-Dichlorphenyl)piperazin-1-yl)butyl-2-benzo[b]tellurophenylcarbamid

### Synthese analog zu Beispiel 7.

### Ausbeute: 92 mg (45 %)

Smp.: 92°C. MS m/z 560 (M⁺). IR (NaCl) : 3288; 2938; 2819; 1651; 1578; 1557; 1448; 1242; 1044; 734. ¹H-NMR (CDCl₃, 360 MHz) δ (ppm): 1,63-1,74 (m, 4H, CH₂-CH₂); 2,48-2,52 (t, J=7,1 Hz, 2H, CH₂N); 2,66 (m, 4H, pip); 3,05 (m, 4 H, pip); 3,46-3,51 (m, 2H, CH₂NCO); 6,70-6,72 (brt, J= 4,8 Hz, 1H, NH); 6,83-6,86 (dd, J=1,8 Hz, J=7,8 Hz, 1H, Phenyl); 7,07-7,22 (m, 3H, Phenyl, H₆); 7,36-7,41 (m, 1 H, H₅); 7,81-7,83 (d, J=7,6 Hz, 1H, H₄); 7,91-7,94 (d, J=7,6 Hz, 1H, H₇); 8,17 (m, 1H, H₃). ¹³C-NMR (CDCl₃, 90 MHz) δ (ppm): 166,1; 151,1; 147,9; 140,2; 135,0; 134,9; 134,0; 132,3; 129,2; 127,5; 127,4; 125,8; 125,5; 124,6; 118,6; 58,0; 53,3; 51,2; 40,4; 27,5; 24,4.

### Beispiel 10

### N-4-(4-(2,3-Dichlorphenyl)piperazin-9-yl)butyl-2-indolylcarbamid

### Synthese analog zu Beispiel 1.

### Ausbeute: 48 mg (25 % Ausbeute über 2 Reaktionsschritte)

Smp.: 148°C. MS m/z 444 (M⁺). IR (NaCl): 3258; 3059; 2938; 2821; 1636; 1577; 1555; 1507; 1448; 1420; 1308; 1241; 1139; 1044; 961; 908; 779; 747; 733; 669; 647. ¹H-NMR (CDCl₃, 360 MHz) δ (ppm): 1,67-1,74 (m, 4H, CH₂-CH₂); 2,47-2,51 (t, J=6,9 Hz, 2H, CH₂N); 2,67 (m, 4H, pip); 3,13 (m, 4H, pip); 3,53-3,55 (m, 2H, CH₂NCO); 6,59-6,66 (brt, J=4,3 Hz, 1H, NHCO); 6,85 (s, 1H, H₃); 6,90-6,93 (m, 1H, Phenyl); 7,07-7,17 (m, 3H, Phenyl, H₅); 7,28-7,30 (m,1H, H₆); 7,43-7,46 (m, 1H, H₇); 7,62-7,65 (m,1H, H₄); 9,56 (s, 1H, NH). ¹³C-NMR (CDCl₃, 90 MHz) δ (ppm): 161,7; 160,4; 151,1; 136,2; 134,0; 130,9; 127,5; 127,4; 121,8; 120,8; 118,5; 111,9; 101,8; 68,2; 57,9; 53,3; 51,1; 39,5; 27,5; 24,3.

### Beispiel 11

### N-4-(4-(2-Methoxyphenyl)piperazin-1-yl)butyl-5-cyan-2-indolylcarbamid

### Synthese analog zu Beispiel 7.

### Ausbeute: 109 mg (42%)

Smp.: 170°C. MS m/z 431 (M⁺). ¹H-NMR (CDCl₃, 360 MHz) δ (ppm): 1,74-1,78 (m, 4H, CH₂-CH₂); 2,54-2,65 (t, J =6,7 Hz, 2H; CH₂N); 2,79 (m, 4H, pip); 3,17 (m, 4H, pip); 3,55-3,59 (m, 2H, CH₂NCO); 3,85 (s, 3H, OCH₃); 6,84-6,87 (d, J=8,5 Hz, 1H, H₅); 6,88-6,90 (m, 3H, Phenyl); 6,99-7,05 (m, 2H, Phenyl, H₄); 7,07-7,12 (brt, J=3,9 Hz, 1H, NHCO); 7,47-7,50 (dd, J=1,4 Hz, J=8,5 Hz, 1H, H₆); 7,52-7,54 (d, J= 8,5 Hz, 1H, H₇); 8,01 (s, 1 H, H₃); 10,14 (s, 1H, NH). ¹³C-NMR (CDCl₃, 90 MHz) δ (ppm): 152,2; 140,7; 137,7; 133,3; 127,8; 127,3; 126,7; 123,4; 121,1; 120,2; 118,3; 111,3; 103,9; 102,9; 57,6; 55,4; 53,5; 50,0; 39,2; 27,0; 24,2.

### Beispiel 12

### N-4-(4-(2-Methoxyphenyl)piperazin-1-yl)butyl-5-brom-2-indolylcarbamid

### Synthese analog zu Beispiel 7.

### Ausbeute: 112 mg (60%)

Smp.: 188°C. ¹H-NMR (CDCl₃, 360 MHz) δ (ppm): 1,64-1,74 (m, 4H, CH₂-CH₂); 2,46-2,51 (t, J=7,1 Hz, 2H, CH₂N); 2,68 (m, 4H, pip); 3,12 (m, 4H, pip); 3,49-3,64 (m, 2H, CH₂NCO); 3,83 (s, 3H, OCH₃); 6,68-6,71 (brt, J=5,3 Hz, 1H, NHCO); 6,76-6,77 (d, J=1,8 Hz, 1H, H₄); 6,85-6,87 (d, J=7,8 Hz, 1H, Phenyl); 6,92-6,93 (d, J= 3,9 Hz, 2H, Phenyl); 6,98-7,03 (m, 1H, Phenyl); 7,31-7,38 (m, 2H, H₆, H₇); 7,76-7,77 (m, 1H, H₃); 9,64 (s, 1H, NH).

### Beispiel 13

### N-4-(4-(2-Methoxyphenyl)piperazin-1-yl)butyl-6-cyan-2-indolylcarbamid

### Synthese analog zu Beispiel 1.

### Ausbeute: 112 mg (63% Ausbeute über 2 Reaktionsschritte)

Smp.: 174°C. MS m/z 431 (M⁺). IR (NaCl): 2940; 2909; 2803; 2753; 2216; 1645; 1548; 1498; 1321; 1237; 1148; 820; 754; 742. ¹H-NMR (CDCl₃, 360 MHz) δ (ppm): 1,67-1,82 (m, 4H, CH₂-CH₂); 2,47-2,51 (t, J=6,7 Hz, 2H, CH₂N); 2,66 (m, 4H, pip); 3,68-3,70 (m, 2H, CH₂NCO); 3,85 (s, 3H, OCH₃); 3,09 (m, 4H, pip); 6,84-6,94 (m, 4H, Phenyl, H₃); 6,99-7,02 (m, 1H, Phenyl); 7,13-7,16 (brt, J= 5,5 Hz, 1H, NHCO); 7,31-7,34 (m, 1H, H₅); 7,67-7,69 (d, J=8,5 Hz, H₄); 7,84 (s, 1H, H₇); 11,22 (s, 1H. NH). ¹³C-NMR (CDCl₃, 90 MHz) δ (ppm): 161,2; 152,2; 141,1; 135,2; 134,3; 130,5; 123,0; 122,9; 122,7; 120,9; 120,2; 118,1; 117,4; 111.2; 106,6; 102,2; 57,8; 55,3; 53,8; 53,4; 50,4; 39,8; 30,1; 27,3; 24,3.
C H N (%): C₂₅H₂₉N₅O₂·1,4 H₂O;
Ber.: C 65,74; H 7,02; N 15,33; Gef.: C 65,98; H 7,30; N 14,87

### Beispiel 14

### N-4-(4-(2,3-Dichlorphenyl)piperazin-1-yl)butyl-5-brom-2-indolylcarbamid

0,24 mmol (58 mg) HATU, 0,24 mmol HOAt (33 mg) und 0,24 mmol (69 mg) der 5-Brom-2-indolcarbonsäure werden bei 0°C in 5 ml DMF gelöst und 0,48 mmol (0,094 ml) DIEA zugegeben. Anschließend wird 0,26 mmol (78 mg) 4-(4-(2,3-Dichloryphenyl)piperazin-1-yl)butylamin in DMF gelöst und bei 0°C zu der Reaktionslösung zugetropft. Nach 3 Stunden wird der Reaktionsansatz in CHCl₃ aufgenommen und mit NaHCO₃-Lösung sowie Wasser gewaschen. Nach Trocknung mit MgSO₄ wird das Lösungsmittel abgedampft und durch Flashchromatographie (SiO₂; CHCl₃:MM, 98:2) gereinigt. Ausbeute: 94 mg (74 %)

MS m/z 524 (M⁺). IR (NaCl): 3234; 2932, 2821; 1637; 1577; 1545; 1282; 1046; 733. ¹H-NMR (CDCl₃, 360 MHz) δ (ppm): 1,57-1,73 (m, 4H, CH₂-CH₂); 2,51 (t, J=6,9 Hz, 2H, CH₂N); 2,66 (m, 4H, pip); 3,07 (m, 4H, pip); 3,51-3,63 (m, 2H, CH₂NCO); 6,64 (brt, J=5,3 Hz, 1H, NHCO); 6,77 (d, J=1,8 Hz, 1H, Phenyl, H₄); 6,90 (dd, J=2,1 Hz, J=7,5 Hz, 1H, Phenyl); 7,10-7,17 (m, 2H, Phenyl); 7,31-7,38 (m, 2H, H₆, H₃); 7,76-7.77 (m, 1H, H₇); 9,68 (s,1 H, NH).

### Beispiel 15

### N-4-(4-(2,3-Dichlorphenyl)piperazin-1-yl)butyl-6-cyan-2-indolylcarbamid

### Synthese analog zu Beispiel 7.

### Ausbeute: 102 mg (59 %)

Smp.: 174°C. MS m/z 470(M⁺). IR (NaCl): 3215; 2926; 2821; 1634; 1570; 1506; 1239; 1034; 734. ¹H-NMR (CDCl₃, 360 MHz) δ (ppm): 1,69-1,82 (m, 4H, CH₂-CH₂); 2,51 (t, J=6,9 Hz, 2H, CH₂N); 2,66 (m, 4H, pip); 3,06 (m, 4H, pip); 3,58-3,63 (m, 2H, CH₂NCO); 6,85 (brt, J= 5,5 Hz, 1H, NHCO); 6,88-6,91 (m, 2H, Phenyl, H₃); 7.09-7,17 (m, 2H, Phenyl); 7,35 (dd, J=1,4 Hz, J=8,2 Hz, 1H, H₅); 7,70 (d, J=8,5 Hz, H4); 7,84 (s, 1H, H₇); 10,65 (s, 1 H, NH).

### Beispiel 18

### N-4-(4-(2-Methoxyphenyl)piperazin-9-yl)butyl-5-cyan-2-benzo[b]thiophenylcarbamid

0,012 mmol (94 mg) HATU und 0,012 mmol (25 mg) der 5-Cyano-2-benzo[b]thiophencarbonsäure (Bridges, A. J. *Tetr. Lett.* **1992**, 7499-7502) werden bei 0°C in 1 ml DMF und 4 ml CH₂Cl₂ gelöst und 0,024 mmol (0,06 ml) DIEA zugegeben. Anschließend wird 0,013 mmol (34 mg) 4-(4-(2-Methoxyphenyl)piperazin-1-yl)butylamin in CH₂Cl₂ gelöst und bei 0°C zu der Reaktionslösung zugetropft. Nach 2 Stunden wird der Reaktionsansatz in CHCl₃ aufgenommen und mit NaHCO₃-Lösung sowie Wasser gewaschen. Nach Trocknung mit MgSO₄ wird das Lösungsmittel abgedampft und durch Flashchromatographie (SiO₂; Methylenchlorid-Methanol: 98-2) gereinigt.
Ausbeute: 60 mg (91%)

Smp.: 147°C. MS m/z 448 (M⁺). IR (KBr): 3336; 2929; 2816; 2225; 1635; 1500; 1240; 1028; 750. ¹H-NMR (CDCl₃, 360 MHz) δ (ppm): 1,73-1,77 (m, 4H, CH₂-CH₂); 2,59 (t, J=6,4 Hz, 2H, CH₂N); 2,78 (m, 4H, pip); 3,14 (m, 4H, pip); 3,49-3,53 (m, 2H, CH₂NCO), 3,85 (s, 3H, OCH₃); 6,84-6,92 (m, 5H, Phenyl, NH); 6,99-7,04 (m, 1H, Phenyl); 7,60 (dd, J=1,4 Hz, J=8,5 Hz, 1H, H₆); 7,88 (s, 1H, H₃); 7,95 (d, J=8,5 Hz, 1H, H₇); 8,12 (d, J=1,1 Hz, 1H, H₄). ¹³C-NMR (CDCl₃, 90 MHz) δ (ppm): 170,3; 152,2; 140,9; 123,1; 120,9; 118,3; 111,2; 77,2; 76,3; 70,3; 69,7; 68,1; 57,9; 55,3; 53,4; 50,1; 39,2; 27,7; 23,8.
C H N (%) C₂₄H₂₅Cl₂N₅O·1H₂O;
Ber.: C 64,35 H 6,48 N 12,01 S 6.87; Gef.: C 64,59 H 6,13 N 11,77 S 6,44.

### Beispiel 19

### N-4-(4-(2,3-Dichlorphenyl)piperazin-1-yl)butyl-5-cyan-2-benzo[b]thiophenylcarbamid

### Synthese analog zu Beispiel 18.

### Ausbeute: 57 mg (96%)

Smp.: 190°C. MS m/z 487 (M⁺). IR (KBr): 3319; 2929; 2819; 2227; 1633; 1560; 1448; 1242; 1045; 755. ¹H-NMR (CDCl₃, 360 MHz) δ (ppm): 1.65-1.76 (m, 4H,CH₂-CH₂); 2.49 (t, J=6.7 Hz, 2H, CH₂N); 2.65 (m, 4H, pip); 3.04 (m, 4H, pip); 3.49-3.55 (m, 2H, CH₂NCO); 6.78 (br. t., J=5.0 Hz, 1H, NH); 6.85 (dd J=1.8 Hz , J=7.8Hz, 1H,Phenyl); 7.09-7.17 (m, 2H, Phenyl); 7.62 (dd, J=1.4 Hz, J=8.5 Hz, 1H, H₆); 7.77 (s, 1H, H₃); 7.95 (d, J=8.2 Hz, H₇); 8.13 (d, J=1.4 Hz, 1H, H₄). ¹³C-NMR (CDCl₃, 90 MHz) δ (ppm): 161,4; 151,1; 144,5; 142,0; 138,8, 134,1; 129,4, 127,9; 127,5, 127,4; 124,7; 123,9; 123,8; 118,9; 118,4; 108,9; 57,9; 53,3; 51,2; 40,2; 27,4; 24,3.
C H N (%) C₂₀H₂₆Cl₂N₄O S·1,46 H₂O
Ber.: C 56,11 H 5,28 N 10,90; Gef.: C 56,51 H 5,06 N 10,45.

### Beispiel 20

### N-4-(4-(2-Methoxyphenyl)piperazin-1-yl)butyl-6-cyan-2-benzo[b]thiophenylcarbamid

### Synthese analog zu Beispiel 18.

### Ausbeute: 30 mg (43%)

Smp.: 124°C. MS m/z 448 (M⁺). IR (KBr): 3290; 2937; 2816; 2225; 1619; 1543; 1500; 1242; 1026; 751. ¹H-NMR (CDCl₃, 360 MHz) δ (ppm): 1,72-1,90 (m, 4H, CH₂-CH₂); 2,90 (t, J=7,3 Hz, 2H, CH₂N); 3,15 (m, 4H, pip); 3,31 (m, 4H, pip); 3,50-3,56 (m, 2H, CH₂NCO), 3,82 (s, 3H, OCH₃); 6.84- 6,92 (m, 3 H, Phenyl); 7,01-7,07 (m, 1H, Phenyl); 7,13 (br,t,, J=5,7, 1 H, NH); 7,53 (dd, J=1,4 Hz, J=8,2 Hz, 1H, H₅); 7,84 (d, J=7,8 Hz, 1 H, H₄); 7,84 (s, 1H, H₃); 8,10-8,11 (m, 1H, H₇). ¹³C-NMR (CDCl₃, 90 MHz) δ (ppm): 161,5; 152,3; 141,9; 141,0; 140,4; 127,5; 127,4, 125,6; 124,2; 123,1, 120,9; 118,8; 118,1; 111,3; 109,5; 77,2; 76,3; 70,3; 69,7; 68,1; 57,9; 55,3; 53,4; 50,4; 40,1; 27,3; 24,2.

### Beispiel 21

### N-4-(4-(2,3-Dichlorphenyl)piperazin-1-yl)butyl-6-cyan-2-benzo[b]thiophenylcarbamid

### Synthese analog zu Beispiel 18.

### Ausbeute: 26 mg (43%)

Smp.: 137°C. MS m/z 486 (M⁺). IR (KBr): 3335; 2933; 2821; 2226; 1638;1544; 1448; 1242; 1044; 735. ¹H-NMR (CDCl₃, 360 MHz) δ (ppm): 1,67-1,74 (m, 4H, CH₂-CH₂); 2,49 (t, J=6,4 Hz, 2H, CH₂N,); 2,65 (m, 4H, pip); 3,04 (m, 4H, pip); 3,49-3,54 (m, 2H, CH₂NCO); 6,75 (br,t, J= 4,1 Hz, 1H, NH); 6,84, (dd, J=1,8 Hz, 7,8 Hz, 1H, Phenyl); 7,08-7,17 (m, 2H, Phenyl); 7,60-7,62 (m, 1H, H₅); 7,78 (s, 1H, H₃); 7,88-7,90 (m, 1H, H₄); 8,19 (br, s, 1H, H₇). ¹³C-NMR (CDCl₃, 90 MHz) δ (ppm): 161,4; 151,1; 143,6; 141,8; 140,3; 134,1; 127,5; 127,4; 125,2; 124,7, 124,2; 118,7; 118,4; 109,5; 109,5; 58,0; 53,3; 51,3; 51,2; 40,3; 27,4; 24,3.
C H N (%): C₂₄H₂₅Cl₂N₅O·H₂O;
Ber.: C 59,02; H 5,57; N 14,34. Gef.: C 58,76; H 5,30; N 14,19.

### Beispiel 22

### N-4-(4-(2-Methoxyphenyl)piperazin-1-yl)butyl-5-brom-2-benzo[b]thiophenylcarbamid

### Synthese analog zu Beispiel 7.

### Ausbeute: 73 mg (58%)

Smp.: 156°C. MS m/z 502 (M⁺). IR (KBr): 3316; 2934; 2821; 1633; 1558; 1500; 1242; 1026; 750. ¹H-NMR (CDCl₃, 360 MHz) δ (ppm): 1,69-1,72 (m, 4H,CH₂-CH₂); 2,57 (t, J=6,5 Hz, 2H, CH₂N); 2,77 (m, 4H, pip); 3,12 (m, 4H, pip); 3,49-3,51 (m, 2H, CH₂NCO); 3,85 (s, 3 H, OCH₃); 6,83-6,91 (m, 4H, Phenyl); 7,01-7,06 (m, 1H, Phenyl); 7,15 (brt, J=4,9 Hz, 1H, NH); 7,42 (dd, J=8,5 Hz, J=1,8 Hz, 1H, H₆); 7,60 (d, J=8,5 Hz, 1H, H₇); 7,73 (s, 1H, H₃); 7,86 (d, J=1,7 Hz, 1H, H₄). ¹³C-NMR (CDCl₃, 90 MHz) δ (ppm): 170,3; 152,2; 140,9; 123,1; 120,9; 118,3; 111,2; 77,2; 76,3; 70,3; 69,7; 68,1; 57,9; 55,3; 53,4; 50,1; 39,2; 27,7; 23,8, CHN : C₂₄H₂₈BrN₃O₂S (%): Ber,: C 57,37 H 5,62 N 8,36 Gef,: C 65,98 H 7,30N 14,87.

### Beispiel 23

### N-4-(4-(2,3-Dichlorphenyl)piperazin-1-yl)butyl-5-brom-2-benzo[b]thiophenylcarbamid

### Synthese analog zu Beispiel 7.

### Ausbeute: 78 mg (60%)

Smp.: 178°C. MS m/z 541 (M⁺). IR (KBr;): 3316; 2929; 2821; 1631; 1560; 1242; 1068; 756. ¹H-NMR (CDCl₃, 360 MHz) δ (ppm): 1,63-1,69 (m, 4H,CH₂-CH₂); 2,48 (t, J=6,7 Hz, 2H, CH₂N); 2,64 (m, 4H, pip); 3,04 (m, 4H, pip); 3,48-3,53 (m, 2H, CH₂NCO); 6,71 (br, t,, J= 5,1 Hz, 1H, NH); 6,83-6,86 (m, 1H, Phenyl); 7,09-7,17 (m, 2H, Phenyl); 7,51(dd, J=1,8 Hz, J=8,5 Hz, 1H, H₆); 7,67 (s, 1H, H₃); 7,72 (d, J=8,5 Hz, 1H, H₇); 7,95 (d, J=1,8 Hz, 1H, H₄). ¹³C-NMR (CDCl₃, 90 MHz) δ (ppm): 161,9; 160,8; 140,7; 140,6, 139,2; 134,1; 129,3; 127,4; 127,3; 124,6; 124,1; 123,8, 118,9; 118,5; 57,9; 55,3; 51,2; 40,2; 27,5; 24,4.
CHN (%): C₂₃H₂₄BrCl₂N₃OS·0,25H₂O;
Ber.: C 50,61 H 4,52 N 7,70 S 5,87; Gef.: C 50,61 H 4,49 N 7,64 S 5.87.

### Beispiel 24

### N-4-(4-(2-Methoxyphenyl)piperazin-1-yl)butyl-2-indolylcarbamid

### Synthese analog zu Beispiel 7.

### Ausbeute: 89 mg (55%)

MS m/z 406 (M⁺). IR (NaCl): 3251; 3055; 2935; 2809; 1639; 1549; 1241; 1016; 746. ¹H-NMR (CDCl₃, 360 MHz) δ (ppm): 1,67-1,74 (m, 4H,CH₂-CH₂); 2,48 (t, J=6,7 Hz, 2H, CH₂N); 2,67 (m, 4H, pip); 3,11 (m, 4H, pip); 3,51-3,56 (m, 2H, CH₂NCO); 3,85 (s, 1H, OCH₃); 6,59 (br, t, J= 4,9 Hz, 1H, NHCO); 6,83-6,94 (m, 3H, Phenyl); 6,97-7,02 (m, 1H, Phenyl); 7,11-7,15 (m, 1H, H₅); 7,25 (s, 1H, H₃); 7,29 (dd, J=1,1 Hz, J=7,1 Hz, 1H, H₆); 7,43 (d, J=8,9 Hz, 1H, H₇); 7,63 (d, J=8,9 Hz, 1H, H₄); 9,50 (s, 1 H).

### Beispiel 25

### N-4-(4-(2,3-Dichlorphenyl)piperazin-1-yl)butyl-5-cyan-2-indolylcarbamid

### Synthese analog zu Beispiel 7.

### Ausbeute: 102 mg (59%)

Smp.: 96°C. MS m/z 470 (M⁺). IR (KBr): 3315; 2926; 2821; 2218, 1634; 1570; 1506; 1239; 1043; 734. ¹H-NMR (CDCl₃, 360 MHz) δ (ppm): 1,69-1,82 (m, 4H,CH₂-CH₂); 2,51 (t, J=6,9 Hz, 2H, CH₂N); 2,66 (m, 4H, pip); 3,06 (m, 4H, pip); 3,58-3,63 (m, 2H, CH₂NCO); 6,85 (br, t" J=5,5 Hz, 1H, NHCO); 6,88-6,91 (m, 2H, Phenyl, H₃); 7,09-7,17 (m, 2H, Phenyl); 7,35 (dd, J=1,4 Hz, J=8,2 Hz, 1H, H₅); 7,70 (d, J=8,5 Hz, H₄); 7,84 (s, 1H, H₇); 10,65 (s, 1H, NH). ¹³C-NMR (CDCl₃, 90 MHz) δ (ppm): 161,2; 151,1; 135,2; 134,3; 134,0; 130,5; 127,5; 127,4; 124,6; 123,1; 122,8; 120,1; 118,5; 117,2; 106,9; 102,0; 58,7; 53,3; 51,2; 41,1; 39,9; 24,4.
C H N (%): C₂₄H₂₅Cl₂N₅O·H₂O;
Ber.: C 59,02; H 5,57; N 14,34. Gef.: C 58,76; H 5,30; N 14.19.

### BIOLOGISCHE AKTIVITÄT

Die biologischen Aktivitäten der erfindungsgemäßen Verbindungen wurden in Radioligandbindungsuntersuchungen ermittelt. Alle Radioligandexperimente wurden nach von uns beschriebenen Methoden durchgeführt (Hübner, H. et al. *J. Med. Chem.* **2000**, *43*, 756-762). Für die Messung der Affinitäten zu den Rezeptoren der D2-Familie kamen Membranhomogenate von chinesischen Hamster-Ovarialzellen (CHO-Zellen) zum Einsatz, die jeweils den humanen D2long-, den humanen D2short- (Hayes, G. et al. *Mol. Endocrinol.* **1992,** *6,* 920-926), den humanen D3- (Sokoloff, P. et al. *Eur. J. Pharmacol.* **1992,** *225,* 331-337) oder den humanen D4.4-Rezeptorsubtyp (Asghari, V. *J. Neurochem.* **1995**, *65*, 1157-1165) stabil exprimierten. Prinzipiell erfolgten die Bindungsassays durch Inkubation der Rezeptorhomogenate mit dem Radioligand [³H] Spiperon und der zu untersuchenden Verbindung in verschiedenen Konzentrationen. Die Ermittlung der Affinitäten zum D1-Rezeptor erfolgte mit nativen Membranhomogenaten, gewonnen aus dem Striatum des Schweines, und dem D1-selektiven Radioliganden [³H]SCH 23390.

Zur Bestimmung der Bindungsstärken der Verbindungen zu den SerotoninRezeptorsubtypen 5-HT1A und 5-HT2 wurden Cortex-Membranpräparationen des Schweines mit den Radioliganden [³H]8-OH-DPAT (für 5-HT1A) oder [³H]Ketanserin (5-HT2) und den Verbindungen inkubiert. Hinweise auf eine gleichzeitige Bindung der Verbindungen an den serotonergen 5-HT2-Rezeptor und an den adrenergen Rezeptorsubtyp α1 bei der Markierung mit dem Radioliganden [³H]Ketanserin wurden in einem Parallelexperiment bei selektiver Blockade des α1-Rezeptors durch Prazosin bestätigt. Somit repräsentieren Ki-Werte, die in Anwesenheit von 10 µM Prazosin ermittelt wurden, die alleinige Bindung an den 5-HT2-Rezeptor. Ergänzend wurde die Affinität zu α1-Rezeptoren des Schweines in einem separaten Experiment mit dem α1-selektiven Radioliganden [³H]Prazosin bestimmt.

Die Ergebnisse der Rezeptorbindungsuntersuchungen an den Dopaminrezeptorsubtypen sind in Tabelle 1 zusammengefasst.

Alle im Bindungsassay untersuchten Verbindungen zeigten dabei gute bis sehr gute Affinitäten zu den Dopaminrezeptoren mit einer klaren Bindungspräferenz zu den Subtypen der D2-Familie. Unabhängig von der Partialstruktur ist dabei immer eine deutliche Selektivität zum D3-Rezeptor zu erkennen. Höchste D3-Affinität kann erreicht werden, wenn als Heteroarenkomponente Benzo[b]thiophen oder Indol eingesetzt wird. So weisen die Verbindungen der Beispiele 1, 2, 10 bis 13 sowie 19 bis 22 hervorragende Ki-Werte zwischen 0,23 und 0,57 nM auf.

Das Substitutionsmuster der Arylpiperazinylkomponente beeinflusst vor allem die Ausprägung der Selektivität der D3-Affinität gegenüber den anderen Rezeptorsubtypen. Die 2,3-dichlorphenylsubstituierten Verbindungen (Beispiele 2, 6 und 10) zeigen mit Selektionskoeffizienten von über 1000 eine bisher noch nicht beschriebene D3-Selektivität bei gleichzeitiger subnanomolarer Affinität. Interessanterweise sind die Ferrocenylderivate der Beispiele 16 und 17 durch eine hohe D4-Affinität charakterisiert, wobei Beispiel 17 mit Ki-Werten von 0,47 nM für den D3-Rezeptor und 0,63 nM für den D4-Rezeptor ein sehr außergewöhnliches Rezeptorbindungsprofil aufweist.

Untersuchungen zur Bestimmung der intrinsischen Aktivität der Beispielverbindungen wurden in einem Mitogeneseassay in Anlehnung an die Literatur (Hübner, H. et al. *J. Med. Chem.* **2000,** *43,* 4563-4569; Löber, S. *Bioorg. Med. Chem. Lett.* **2002,** 12.17, 2377-2380) durchgeführt. Dabei zeigte sich exemplarisch für die Verbindung aus Beispiel 1 für den D3-Rezeptor eine partialagonistische Aktivität von 49 % der maximalen Rezeptorstimulation, die durch den vollen Agonisten Quinpirol als Referenzverbindung ausgelöst werden kann. Kurvenberechnung dieser Konzentrations-Wirkungs-Untersuchung ergab dabei einen EC₅₀-Wert von 0,38 nM.

**Tabelle 1: Bindungsdaten und Selektivitätsmuster der Verbindungen von Formel (I) bis (IV) für die Dopamin-Rezeptoren pD1, hD2long, hD2short, hD3 und hD4.4**

| Ki-Werte in [nM]^{a} | | | | | | D3-Selektivität | | |
|---|---|---|---|---|---|---|---|---|
| Verbindung | | | | | | D2long/D3 | D2short/D3 | D4.4/D3 |
| | D1 | D2long | D2short | D3 | D4.4 | | | |
| Beispiel 1 | 670 | 87 | 52 | 0,23 | 15 | 380 | 230 | 65 |
| Beispiel 2 | 8800 | 3300 | 2600 | 0,5 | 340 | 6600 | 5200 | 680 |
| Beispiel 3 | 1100 | 110 | 84 | 1,1 | 30 | 100 | 76 | 27 |
| Beispiel 4 | 2900 | 320 | 80 | 1,2 | 93 | 270 | 67 | 78 |
| Beispiel 5 | 590 | 96 | 61 | 0,69 | 17 | 140 | 88 | 25 |
| Beispiel 6 | 21000 | 10000 | 4800 | 3,4 | 3100 | 2900 | 1400 | 910 |
| Beispiel 7 | 1400 | 130 | 89 | 4,2 | 57 | 31 | 21 | 14 |
| Beispiel 8 | 380 | 63 | 39 | 0,72 | 35 | 88 | 54 | 49 |
| Beispiel 9 | 1400 | 91 | 48 | 0,55 | 150 | 170 | 87 | 270 |
| Beispiel 10 | 11000 | 3100 | 1600 | 0,56 | 1700 | 5500 | 2900 | 3000 |
| Beispiel 11 | 920 | 140 | 99 | 0,57 | 24 | 250 | 180 | 44 |
| Beispiel 12 | 390 | 110 | 44 | 0,24 | 16 | 460 | 180 | 67 |
| Beispiel 13 | 460 | 160 | 100 | 0,25 | 40 | 640 | 400 | 160 |
| Beispiel 14 | 4200 | 2300 | 770 | 0,73 | 600 | 3200 | 1100 | 820 |
| Beispiel 15 | 17000 | 340 | 110 | 0,35 | 630 | 970 | 310 | 1800 |
| Beispiel 18 | 430 | 68 | 39 | 0,46 | 45 | 150 | 85 | 98 |
| Beispiel 19 | 1700 | 410 | 310 | 0,25 | 650 | 1600 | 1200 | 2600 |
| Beispiel 20 | 1100 | 210 | 130 | 0,33 | 37 | 640 | 390 | 110 |
| Beispiel 21 | 1700 | 180 | 60 | 0,26 | 72 | 690 | 230 | 280 |
| Beispiel 22 | 550 | 49 | 30 | 0,26 | 58 | 190 | 120 | 220 |
| Beispiel 23 | 4700 | 1700 | 970 | 3,2 | 1700 | 1500 | 300 | 530 |
| Beispiel 24 | 1200 | 200 | 160 | 0,70 | 40 | 290 | 230 | 57 |
| Beispiel 25 | 1700 | 140 | 27 | 0,91 | 210 | 150 | 30 | 230 |

| | | | | | | | | |
|---|---|---|---|---|---|---|---|---|
| ^{a} Durchschnittswerte aus 2-9 Einzelexperimenten durchgeführt als Triplikate | | | | | | | | |

Die Untersuchung der Affinitäten zu den Serotoninrezeptorsubtypen 5-HT1A und 5-HT2 sowie zum adrenergen Rezeptor α1 sind in Tabelle 2 beschrieben. Dabei ist unabhängig von den Partialstrukturen der Derivate eine bevorzugte Bindung an den 5-HT1A-Subtyp im Vergleich zu 5-HT2 zu erkennen. Die Verbindungen der Beispiele 1, 3, 7, 8 und 16 sind mit gemessenen Ki-Werten von 8 bis 19 nM durch eine hohe Affinität zum α1-Rezeptor charakterisiert.

Struktur-Wirkungs-Überlegungen zeigen für die Bindung an diese Rezeptoren eine deutliche Abhängigkeit vom Substitutionsmuster der Arylpiperazinylpartialstruktur. Wie bei den Dopaminrezeptoren geht bei den Derivaten mit 2,3-Dichlorphenylrest die Bindung zu den 5-HT- und zum α1-Rezeptor deutlich zurück, was zu einer Erweiterung des Selektivitätsspektrums gegenüber der D3-Rezeptoraffinität dieser Verbindungen führt.

**Tabelle 2: Bindungsdaten der Substanzen von Formel (I) bis (IV) für die Serotonin-Rezeptoren p5-HT1A. p5-HT2 sowie für den adrenergen Rezeptorsubtyp pα1**

| Verbindungen | Ki-Werte in [nM]^{a} | | | |
|---|---|---|---|---|
| | 5-HT1A | 5-HT2^{b} | α1^{c} | α1^{d} |
| Beispiel 1 | 41 | 350 | 15 | 6,4 |
| Beispiel 2 | 360 | 2000 | --- | 370 |
| Beispiel 3 | 17 | 660 | 14 | 3,3 |
| Beispiel 4 | 480 | 11000 | --- | 160 |
| Beispiel 5 | 68 | 140 | --- | 5,3 |
| Beispiel 6 | 2500 | 540 | --- | 1300 |
| Beispiel 7 | 37 | 390 | 8,2 | 11 |
| Beispiel 8 | 69 | 420 | 15 | 3,5 |
| Beispiel 9 | 130 | 730 | --- | 100 |
| Beispiel 10 | 610 | 1700 | --- | 220 |
| Beispiel 11 | 83 | 440 | 24 | 5,9 |
| Beispiel 12 | 440 | 280 | --- | 6,4 |
| Beispiel 13 | 47 | 220 | --- | 4,3 |
| Beispiel 14 | 1600 | 690 | --- | 500 |
| Beispiel 15 | 390 | 320 | --- | 2000 |
| Beispiel 18 | 54 | 580 | --- | 2,9 |
| Beispiel 19 | 190 | 280 | --- | 230 |
| Beispiel 20 | 71 | 660 | --- | 8.3 |
| Beispiel 21 | 110 | 290 | --- | 45 |
| Beispiel 22 | 180 | 760 | --- | 2,5 |
| Beispiel 23 | 430 | 14000 | --- | 320 |
| Beispiel 24 | 32 | 420 | 11 | 7,3 |
| Beispiel 25 | 190 | 220 | -- | 220 |

| | | | | |
|---|---|---|---|---|
| ^{a} Durchschnittswerte aus 2-6 Einzelexperimenten durchgeführt mit Triplikaten ^{b} Ki-Wert bei gleichzeitiger Inkubation mit 10 µM Prazosin ^{c} Ki-Wert abgeleitet von der hochaffinen Bindungsstelle bei Markierung mit [³H]Ketanserin ^{d} Ki-Wert aus dem Kompetitionsexperiment mit dem α1-selektiven Radioliganden [³H]Prazosin | | | | |

## Patentansprüche

1. Verbindung, die aus einer der folgenden ausgewählt ist:
N-4-(4-(2-Methoxyphenyl)piperazin-1-yl)butyl-5-cyan-2-benzo[b]thiophenylcarbamid,
N-4-(4-(2,3-Dichlorphenyl)piperazin-1-yl)butyl-5-cyan-2-benzo[b]thiophenylcarbamid,
N-4-(4-(2-Methoxyphenyl)piperazin-1-yl)butyl-6-cyan-2-benzo[b]thiophenylcarbamid,
N-4-(4-(2,3-Dichlorphenyf)piperazin-1-yl)butyl-6-cyan-2-benzo[b]thiophenylcarbamid,
N-4-(4-(2-Methoxyphenyl)piperazin-1-yl)butyl-2-benzo[b]thiophenylcarbamid,
N-4-(4-(2,3-Dichlorphenyl)piperazin-1-yl)butyl-2-benzo[b]thiophenylcarbamid,
N-4-(4-(2-Methoxyphenyl)piperazin-1-yl)butyl-5-brom-2-benzo[b]thiophenylcarbamid,
N-4-(4-(2,3-Dichlorhenyl)piperazin-1-yl)butyl-5-brom-2-benzo[b]thiophenylcarbamid,
N-4-(4-(2,3-Dichlorphenyl)piperazin-1-yl)butyl-2-indolylcarbamid,
N-4-(4-(2-Methoxyphenyl)piperazin-1-yl)butyl-5-cyan-2-indolylcarbamid,
N-4-(4-(2-Methoxyphenyl)piperazin-1-yl)butyl-5-brom-2-indolylcarbamid,
N-4-(4-(2-Methoxyphenyl)piperazin-1-yl)butyl-6-cyan-2-indolylcarbamid,
N-4-(4-(2,3-Dichlorphenyl)piperazin-1-yl)butyl-5-brom-2-indolylcarbamid,
N-4-(4-(2,3-Dichlorphenyl)piperazin-1-yl)butyl-6-cyan-2-indolylcarbamid,
N-4-(4-(2,3-Dichlorphenyl)piperazin-1-yl)butyl-5-cyan-2-indolylcarbamid,
N-4-(4-(2-Methoxyphenyl)piperazin-1-yl)butyl-5-cyan-2-benzo[b]furanylcarbamid,
N-4-(4-(2-Methoxyphenyl)piperazin-1-yl)butyl-2-benzo[b]furanylcarbamid,
N-4-(4-(2,3-Dichlorphenyl)piperazin-1-yl)butyl-2-benzo[b]furanylcarbamid,
N-4-(4-(2-Methoxyphenyl)piperazin-1-yl)butyl-5-brom-benzo[b]furanylcarbamid,
N-4-(4-(2,3-Dichlorphenyl)piperazin-1-yl)butyl-5-brom-2-benzo[b]furanylcarbamid,
N-4-(4-(2-Methoxyphenyl)piperazin-1-yl)butyl-2-benzo[b]tellurophenylcarbamid und
N-4-(4-(2,3-Dichlorphenyl)piperazin-1-yl)butyl-2-benzo[b]tellurophenylcarbamid,
oder ein pharmazeutisch akzeptables Salz davon.

2. Arzneimittel, das eine oder mehrere der Verbindungen gemäß Anspruch 1 enthält.

3. Arzneimittel nach Anspruch 2, das zusätzlich L-Dopa zur gleichzeitigen oder zeitlich aufeinanderfolgenden Verabreichung an den Patienten enthält.

4. Verwendung einer Verbindung nach Anspruch 1 zur Herstellung eines Arzneimittels zur Therapie oder Prophylaxe von Kokain-, Alkohol-, Opiat- und Nikotinsucht; neurodegenerativen Störungen, insbesondere Morbus Parkinson; sexueller Dysfunktion; Depression oder Schizophrenie.

5. Verwendung einer Verbindung nach Anspruch 1 zur Herstellung eines Arzneimittels zur Therapie oder Prophylaxe von Hyperprolaktinämie, Hyperprolaktinom, Glaucoma, kognitiven Störungen, Restless Leg-Syndrom, Hyperaktivitätssyndrom (ADHS), Parkinson-assoziierten Bewegungsstörungen, L-Dopa-induzierten Störungen, Segawa-Syndrom, tardiven Bewegungsstörungen und zum Medikamenten-unterstützten Abstillen nach Schwangerschaften.

6. Verwendung nach Anspruch 5, wobei das Arzneimittel zur Therapie oder Prophylaxe von Segawa-Syndrom, spontaner Parkinson-assoziierter Dyskinesie oder Dystonie oder tardiver oder L-Dopa-induzierter Dyskinesie oder Dystonie vorgesehen ist.

7. Verfahren zur Herstellung einer Verbindung wie in Anspruch 1 definiert, umfassend das Umsetzen einer Verbindung der allgemeinen Formel (A) in aktivierter Form, insbesondere in Form des Carbonsäurehalogenids: 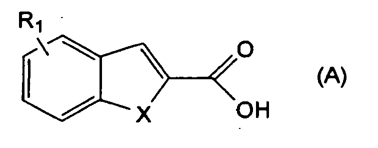 mit einer Verbindung der allgemeinen Formel (B): 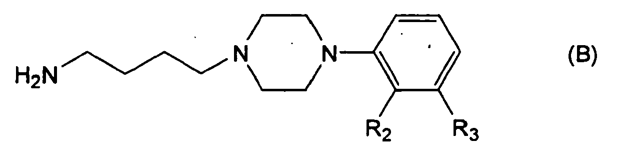 worin R₁, R₂, R₃ und X gemäß den Verbindungen nach Anspruch 1 definiert sind.

## Claims

1. Compound which is selected from one of the following:
N-4-(4-(2-methoxyphenyl)piperazin-1-yl)butyl-5-cyano-2-benzo[b]thiophenyl carbamide,
N-4-(4-(2,3-dichlorophenyl)piperazin-1-yl)butyl-5-cyano-2-benzo[b]thiophenyl carbamide,
N-4-(4-(2-methoxyphenyl)piperazin-1-yl)butyl-6-cyano-2-benzo[b]thiophcnyl carbamide,
N-4-(4-(2,3-dichlorophenyl)piperazin-1-yl)butyl-6-cyano-2-benzo[b]thiophenyl carbamide,
N-4-(4-(2-methoxyphenyl)piperazin-1-yl)butyl-2-benzo[b]thiophenyl carbamide,
N-4-(4-(2,3-dichlorophenyl)piperazin-1-yl)butyl-2-benzo[b]thiophenyl carbamide,
N-4-(4-(2-methoxyphenyl)piperazin-1-yl)butyl-5-bromo-2-benzo[b]thiophenyl carbamide,
N-4-(4-(2,3-diehlorophenyl)piperazin-1-yl)butyl-5-brorno-2-benzo[b]thiophenyl carbamide,
N-4-(4-(2,3-dichlorophenyl)piperazin-1-yl)butyl-2-indolyl carbamide,
N-4-(4-(2-methoxyphenyl)piperazin-1-yl)butyl-5-cyano-2-indolyl carbamide,
N-4-(4-(2-methoxyphenyl)piperazin-1-yl)butyl-5-bromo-2-indolyl carbamide,
N-4-(4-(2-niethoxyphenyl)piperazin-1-yl)butyl-6-cyano-2-indolyl carbamide,
N-4-(4-(2,3-dichlorophenyl)piperazin-1-yl)butyl-5-bromo-2-indolyl carbamide,
N-4-(4-(2,3-dichlorophenyl)piperazin-1-yl)butyl-6-cyano-2-indolyl carbamide,
N-4-(4-(2,3-dichlorophenyl)piperazin-1-yl)butyl-5-cyano-2-indolyl carbamide,
N-4-(4-(2-methoxyphenyl)piperazin-1-yl)butyl-5-cyano-2-benzo[b]furanyl carbamide.
N-4-(4-(2-methoxyphenyl)piperazin-1-yl)butyl-2-benzo[b]furanyl carbamide,
N-4-(4-(2,3-dichlorophenyl)piperazin-1-yl)butyl-2-benzo[b]furanyl carbamide,
N-4-(4-(2-methoxyphenyl)piperazin-1-yl)butyl-5-bromo-benzo[b]furanyl carbamide,
N-4-(4-(2,3-dichlorophenyl)piperazin-1-yl)butyl-5-bromo-2-benzo[b]furanyl carbamide,
N-4-(4-(2-methoxyphenyl)piperazin-1-yl)butyl-2-benzo[b]tellurophenyl carbamide and
N-4-(4-(2,3-dichlorophenyl)piperazin-1-yl)butyl-2-benzo[b]tellurophenyl carbamide
or a pharmaceutically acceptable salt thereof.

2. Medicament which contains one or more of the compounds according to claim 1.

3. Medicament according to claim 2, which additionally contains L-dopa for simultaneous or temporally sequential administration to the patient.

4. Use of a compound according to claim 1 for producing a medicament for the treatment or prophylaxis of cocaine, alcohol, opiate and nicotine addition; neurodegenerative disturbances, in particular Parkinson's disease; sexual dysfunction; depression or schizophrenia.

5. Use of a compound according to claim 1 for producing a medicament for the treatment or prophylaxis of hyperprolactinaemia, hyperprolactinoma, glaucoma, cognitive disturbances, restless leg syndrome, hyperactivity syndrome (ADHS), Parkinson-associated motor disturbances, L-dopa-induced disturbances, Segawa syndrome, tardive motor disturbances and for medicament-assisted weaning after pregnancies.

6. Use according to claim 5, wherein the medicament is intended for the treatment or prophylaxis of Segawa syndrome, spontaneous Parkinson-associated dyskinesia or dystonia or tardive or L-dopa-induced dyskinesia or dystonia.

7. Process for producing a compound as defined in claim 1, comprising the reaction of a compound of the general formula (A) in active form, in particular in the form of the carboxylic acid halide: 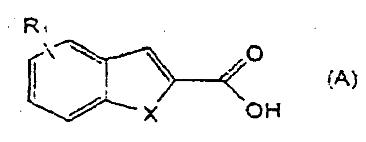 with a compound of the general formula (B): 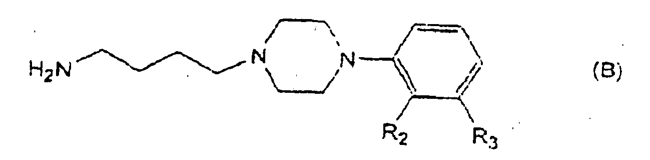 wherein R₁, R₂, R₃ and X are defined according to the compounds of claim 1.

## Revendications

1. Composé choisi dans le groupe suivant :
N-4-(4-(2-méthoxyphényl)pipérazin-1-yl)butyl-5-cyan-2-benzo[b]thiophénylcarbamide,
N-4-(4-(2,3-dichlorophényl)pipérazin-1-yl)butyl-5-cyan-2-benzo[b]thiophénylcarbamide,
N-4-(4-(2-méthoxyphényl)pipérazin-1-yl)butyl-6-cyan-2-benzo[b]thiophénylcarbamide,
N-4-(4-(2,3-dichlorophényl)pipérazin-1-yl)butyl-6-cyan-2-benzo[b]thiophénylcarbamide,
N-4-(4-(2-méthoxyphényl)pipérazin-1-yl)butyl-2-benzo[b]thiophénylcarbamide,
N-4-(4-(2,3-dichlorophényl)pipérazin-1-yl)butyl-2-benzo[b]thiophénylcarbamide,
N-4-(4-(2-méthoxyphényl)pipérazin-1-yl)butyl-5-bromo-2-benzo[b]thiophénylcarbamide,
N-4-(4-(2,3-dichlorophényl)pipérazin-1-yl)butyl-5-bromo-2-benzo[b]thiophénylcarbamide,
N-4-(4-(2,3-dichlorophényl)pipérazin-1-yl)butyl-2-indolylcarbamide,
N-4-(4-(2-méthoxyphényl)pipérazin-1-yl)butyl-5-cyan-2-indolylcarbamide,
N-4-(4-(2-méthoxyphényl)pipérazin-1-yl)butyl-5-bromo-2-indolylcarbamide,
N-4-(4-(2-méthoxyphényl)pipérazin-1-yl)butyl-6-cyan-2-indolylcarbamide,
N-4-(4-(2,3-dichlorophényl)pipérazin-1-yl)butyl-5-bromo-2-indolylcarbamide,
N-4-(4-(2,3-dichlorophényl)pipérazin-1-yl)butyl-6-cyan-2-indolylcarbamide,
N-4-(4-(2,3-dichlorophényl)pipérazin-1-yl)butyl-5-cyan-2-indolylcarbamide,
N-4-(4-(2-méthoxyphényl)pipérazin-1-yl)butyl-5-cyan-2-benzo[b]furanylcarbamide,
N-4-(4-(2-méthoxyphényl)pipérazin-1-yl)butyl-2-benzo[b]furanylcarbamide,
N-4-(4-(2,3-dichlorophényl)pipérazin-1-yl)butyl-2-benzo[b]furanylcarbamide,
N-4-(4-(2-méthoxyphényl)pipérazin-1-yl)butyl-5-bromo-benzo[b]furanylcarbamide,
N-4-(4-(2,3-dichlorophényl)pipérazin-1-yl)butyl-5-bromo-2-benzo[b]furanylcarbamide, N-4-(4-(2-méthoxyphényl)pipérazin-1-yl)butyl-2-benzo[b]tellurophénylcarbamide et
N-4-(4-(2,3-dichlorophényl)pipérazin-1-yl)butyl-2-benzo[b]tellurophénylcarbamide,
ou un sel pharmaceutiquement acceptable de ceux-ci.

2. Médicament contenant un ou plusieurs des composés selon la revendication 1.

3. Médicament selon la revendication 2, contenant en outre de la L-dopa en vue d'une administration simultanée ou consécutive au patient.

4. Utilisation d'un composé selon la revendication 1 pour la fabrication d'un médicament destiné au traitement ou à la prévention de la dépendance à la cocaïne, à l'alcool, aux opiacés et à la nicotine, des troubles neurodégénératifs, en particulier de la maladie de Parkinson, des troubles des fonctions sexuelles, de la dépression et de la schizophrénie.

5. Utilisation d'un composé selon la revendication 1 pour la fabrication d'un médicament destiné au traitement ou à la prévention de l'hyperprolactinémie, des prolactinomes, du glaucome, des troubles cognitifs, du syndrome des jambes sans repos, du syndrome d'hyperactivité (THDA), des troubles moteurs associés à la maladie de Parkinson, des troubles induits par la L-dopa, du syndrome de Segawa, des dyskinésies tardives, ainsi que pour l'aide médicamenteuse à l'arrêt de la lactation après la grossesse.

6. Utilisation selon la revendication 5, dans laquelle le médicament est destiné au traitement ou à la prévention du syndrome de Segawa, des dyskinésies ou dystonies spontanées associées à la maladie de Parkinson ou des dyskinésies ou dystonies induites par la L-dopa.

7. Procédé pour la fabrication d'un composé selon la revendication 1, comprenant la transformation d'un composé selon la formule générale (A) sous une forme activée, en particulier sous la forme de l'halogénure d'acide carbonique : 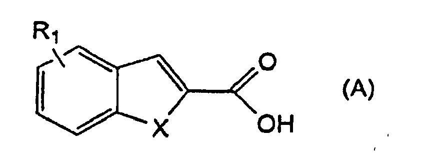 avec un composé selon la formule générale (B) : 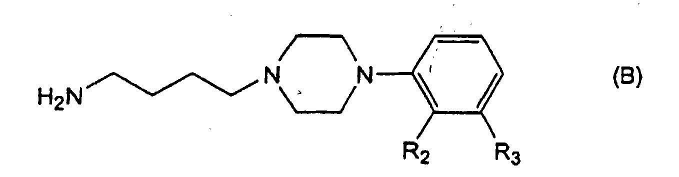 où R₁, R₂, R₃ et X sont définis selon les composés de la revendication 1.
